# EUROPEAN PATENT SPECIFICATION

(11) **EP 3 331 505 B1**
(45) Date of publication and mention of the grant of the patent: **15.07.2020**
(21) Application number: 16742161.9
(22) Date of filing: 07.07.2016
(51) Int. Cl.: A61K 31/517, A61K 9/24, A61K 9/20, A61K 9/28, A61K 31/519, A61P 25/18

(54) **PHARMACEUTICAL COMPOSITION COMPRISING AN ATYPICAL ANTIPSYCHOTIC AGENT AND METHOD FOR THE PREPARATION THEREOF**
PHARMAZEUTISCHE ZUSAMMENSETZUNG MIT EINEM ATYPISCHEN ANTIPSYCHOTISCHEN WIRKSTOFF UND VERFAHREN ZUR HERSTELLUNG DAVON
COMPOSITION PHARMACEUTIQUE COMPRENANT UN AGENT ANTIPSYCHOTIQUE ATYPIQUE ET SON PROCÉDÉ DE PRÉPARATION

(30) Priority: 06.08.2015 GR 20150100356
(43) Date of publication of application: 13.06.2018
(73) Proprietor: Pharmathen S.A., 15351 Pallini Attikis (GR)
(72) Inventor: KARAVAS, Evamgelos, 153 51 Pallini Attikis (GR); KOUTRIS, Efthymios, 153 51 Pallini Attikis (GR); SAMARA, Vasiliki, 153 51 Pallini Attikis (GR); KOUTRI, Ioanna, 153 51 Pallini Attikis (GR); KALASKANI, Anastasia, 153 51 Pallini Attikis (GR); KALANTZI, Lida, 153 51 Pallini Attikis (GR); ABATZIS, Morfis, 153 51 Pallini Attikis (GR); KIZIRIDI, Christina, 153 51 Pallini Attikis (GR)
(86) International application number: PCT/EP2016/001164
(87) International publication number: WO 2017/020984

(56) References cited:
- WO-A2-2011/045774
- US-A1- 2013 034 605
- US-A1- 2013 064 889

## Description

### TECHNICAL FIELD OF THE INVENTION

The present invention relates to oral dosage forms for the controlled delivery of an atypical antipsychotic agent such as Paliperidone or pharmaceutical acceptable salt and a method for the preparation thereof.

### BACKROUND OF THE INVENTION

Antipsychotics are the mainstay of treatment of schizophrenia. Conventional antipsychotics, typified by haloperidol, have a proven track record over the last half-century in the treatment of schizophrenia. While these drugs are highly effective against the positive, psychotic symptoms of schizophrenia, they show little benefit in alleviating negative symptoms or the cognitive impairment associated with the disease.

Second generation antipsychotics such as Paliperidone, also called atypical antipsychotics, differ considerably in their chemical, pharmacological, and clinical profiles and are generally characterized by effectiveness against both positive and negative symptoms associated with schizophrenia and by enhanced safety profile with respect to extrapyramidal symptoms.

Paliperidone also known as 9-hydroxyrisperidone is the major metabolite of risperidone. It shares the characteristic serotonin (5HT_{2A}) and dopamine (D₂) antagonism and receptor binding profile of its parent risperidone. It binds also to a₁-adrenergic receptors, and, with lower affinity, to H₁-histaminergic and a₂-adrenergic receptors, which may explain some of the other effects of Paliperidone.

The chemical name of Paliperidone is 3-[2-[4-(6-fluoro-1,2-benzisoxazol-3-yl)-1-piperidinyl]ethyl]-6,7,8,9-tetrahydro-9-hydroxy-2-methyl-4H-pyrido[1,2-a]pyrimidin-4-one. The molecular formula is C₂₃H₂₇FN₄O₃ corresponding to a molecular weight of 426.48. It is a white to yellow color, non-hygroscopic powder. Paliperidone is sparingly soluble in dichloromethane, slightly soluble in 0.1N HCl and insoluble in water.

WO A-2007/044234 discloses an osmotic dosage form comprising a semi-permeable membrane, a first and a second orifice in the semi-permeable membrane, a controlled release drug layer, a push layer, a fast release drug layer and a barrier layer.

EP B 1539115 discloses a dosage form comprising two or more layers, said first layer comprises Paliperidone, said second layer comprises a polymer, an outer wall surrounding said two or more layers and an orifice in said outer wall.

EP B 2079446 discloses Paliperidone extended release tablet in the form of an inlay tablet comprising an inlay core comprising non-coated Paliperidone and at least one polymer capable of delaying the release of Paliperidone from the inlay core and capable of swelling upon hydration and an outer layer that partially surrounds the inlay core.

US patent application publication No. US 2013/034605 discloses an extended release pharmaceutical composition comprising a core and a multi-layer coating surrounding the core. The core comprises paliperidone or pharmaceutically acceptable salts thereof and one or more pharmaceutical excipients such as binders, fillers or diluents, lubricants, glidants, disintegrants and antioxidants. The multi-layer coating surrounding the core comprises a controlled release layer comprising one or more hydrophoblic agents and one or more hydrophilic agents together with one or more pH dependent polymer layers.

Although each of the patents above represents an attempt to provide dosage forms for the sustained delivery of Paliperidone, there still exists a need for alternative means of controlling delivery that would enhance the initial tolerability and permit initiation of treatment at an effective dose without the need for initial dose titration.

### SUMMARY OF THE INVENTION

It is, therefore, an object of the present invention to provide a stable oral solid dosage formulation comprising an atypical antipsychotic agent and in particular Paliperidone or pharmaceutical acceptable salt thereof, as an active ingredient, providing a uniform and constant rate of release over an extended period of time.

It is another object of the present invention to provide a controlled release pharmaceutical composition for oral administration comprising Paliperidone as an active ingredient, which is bioavailable, with sufficient self-life and good pharmacotechnical properties.

A major object of the present invention is to provide a matrix controlling core comprising specific quantity of hydrophilic/swelling polymer and insoluble/hydrophobic polymer.

An essential object of the present invention is to choose a coating technology which in combination with matrix controlling core will allow drug release with zero order kinetics.

A further approach of the present invention is to provide a controlled release dosage form containing Paliperidone which is manufactured through a fast, simple and cost-effective process.

In accordance with the above objects of the present invention, an oral multi-layered tablet according to the claims for the controlled delivery of Paliperidone is provided comprising a matrix core, an insoluble coating layer and an enteric coating layer in order to achieve zero order drug release.

According to another embodiment of the present invention, a process according to the claims for the preparation of a controlled release pharmaceutical composition of Paliperidone for oral administration is provided comprising a matrix core, an insoluble coating layer and an enteric coating layer in order to achieve zero order drug release is provided, which comprises:
- Mixing of Paliperidone with excipients of matrix core;
- Kneading with solution of ethanol/water;
- Drying of the granules;
- Lubrication of the granules;
- Compression of granules into tablets;
- Applying a separating coating;
- Applying an insoluble coating layer containing also a small amount of active ingredient;
- Applying a separating coating;
- Applying an enteric coating layer.

Other objects and advantages of the present invention will become apparent to those skilled in the art in view of the following detailed description.

### DETAILED DESCRIPTION OF THE INVENTION

For the purposes of the present invention, a pharmaceutical composition comprising an active ingredient (e.g. Paliperidone) is considered to be "stable" if said ingredient degrades less or more slowly than it does on its own and/or in known pharmaceutical compositions.

As already mentioned the main object of the present invention is to provide a controlled release composition of Paliperidone or pharmaceutical acceptable salt thereof that is simple to manufacture, bioavailable, cost effective, stable and possesses good pharmacotechnical properties.

Paliperidone exhibits polymorphism. Two polymorphs are observed, polymorph I and II, in addition to a hydrate and a solvate. Polymorph I is used in the present invention as it is the thermodynamically stable crystal form.

The development of the solid dosage form of Paliperidone SR is based on combination of matrix controlling core and coating technology.

The matrix core tablets contain both hydrophilic/swelling polymer and hydrophobic/pH independent polymer commonly used as matrix forming materials for extended release formulations.

Hydrophilic matrices are the most typical oral extended-release systems because of their ability to provide desired release profiles. Commonly used hydrophilic matrices are selected from hydroxypropylmethylcellulose (HPMC), hydroxypropylcellulose (HPC), hydroxyethylcellulose (HEC), carrageenan, sodium alginate, xanthan gum. Most preferably HPMC K100M is used in the present invention. Hydrophobic materials are also used usually in conjunction with hydrophilic systems. Commonly used hydrophobic matrices are selected from ethyl cellulose, polyvinyl alcohol, HPMC acetate succinate, methacrylic acid copolymers containing low level of quaternary ammonium groups. Most preferably Eudragit® RS is used in the present invention.

It has been surprisingly found that when hydrophilic and hydrophobic polymers are incorporated together in certain quantities in the tablet matrix of the present invention zero order release can be achieved in conjunction with appropriate coating technology. More particularly, the target is achieved when 20-40% by weight of hydrophilic polymer and 5-10% by weight of hydrophobic polymer is comprised in tablet matrix of Paliperidone compositions. Most preferably 40% by weight of hydrophilic polymer and 5% by weight of hydrophobic polymer is comprised in tablet matrix of the preferred composition of the present invention.

The mechanism of drug release from hydrophilic matrix tablets is mainly based on the diffusion of the drug through the hydrated portion of the matrix as well as on the erosion of the outer hydrated polymer on the surface of the matrix. The overall drug release is affected by the rate of water uptake and the diffusion rate of the drug through the swollen gel. Incorporation of a water-insoluble hydrophobic polymer may circumvent the burst release as the former decrease the water penetration in the matrix providing appropriate drug diffusion decrease.

The drug release of the above matrix system is controlled by an insoluble coating layer of hydrophobic polymer in order to achieve zero-order drug release in small intestine and colon. Most preferably hydrophobic polymers are methacrylic acid copolymers containing high level of quaternary ammonium groups such as Eudragit® RL. Such insoluble coating layer is comprised in amount of 1-2% by weight of the tablet core. Moreover, appropriate dissolution profile is achieved when an amount of 10-25% of the labelled amount of Paliperidone is contained in such insoluble coating layer.

In addition, the use of an anionic pH-dependent polymer as enteric coating layer is also necessary in order to minimize drug release in the stomach and create an initial lag phase. Most preferably, a copolymer derived from methacrylate acid/ethyl acrylate, such as Kollicoat® MAE 100 is used as enteric polymer.

A separating layer is applied between tablet core and insoluble coating layer to improve stability by inhibiting any interaction between drug and insoluble coating and between insoluble coating layer and enteric layer so that dissolution of insoluble coating layer begins after dissolution of the enteric coating layer. Typical examples of substances that can be used in the separating layer include sugars, celluloses and cellulose derivatives such as hydroxypropylmethylcellulose, hydroxypropylcellulose, ethylcellulose. Most preferably, hydroxypropylmethylcellulose (Opadry®) is used as separating layer in the compositions of the present invention.

By controlling the amount of each individual excipient in the formulations of the present invention, thus the extend of its contribution in sustained release properties, it is provided a final product in which the in vitro dissolution profile of Paliperidone will be segmented to achieve minimum release in the upper GI tract where solubility and permeability are high, about half of the amount released after 12 hours and maximum release over 24 hours since absorption is much decreased in the colon resulting thus in that zero order drug release .

A number of controlled release compositions comprising different excipients were tested as presented in the following examples to achieve the optimal properties with respect to the objectives of the present invention.

### EXAMPLES

**Table 1: Compositions 1A to 1J (not within claimed scope)**

| **Ingredients** | **Trial 1A** | **Trial 1B** | **Trial 1C** | **Trial 1D** | **Trial 1E** | **Trial 1F** | **Trial 1G** | **Trial 1H** | **Trial 1J** |
|---|---|---|---|---|---|---|---|---|---|
| **Internal phase** | % | | | | | | | | |
| Paliperidone | 0,60 | 0,60 | 0,60 | 0,60 | 0,60 | 0,60 | 0,60 | 0,60 | 0,60 |
| Microcrystalline cellulose | 75,35 | 65,35 | 55,35 | 55,35 | 45,35 | 35,35 | 45,35 | 35,35 | 25,35 |
| HPMC K100M | 20,00 | 20,00 | 20,00 | 40,00 | 40,00 | 40,00 | 50,00 | 50,00 | 50,00 |
| Eudragit® RS PO | - | 10,00 | 20,00 | - | 10,00 | 20,00 | - | 10,00 | 20,00 |
| SLS | 3,00 | 3,00 | 3,00 | 3,00 | 3,00 | 3,00 | 3,00 | 3,00 | 3,00 |
| BHT | 0,05 | 0,05 | 0,05 | 0,05 | 0,05 | 0,05 | 0,05 | 0,05 | 0,05 |

| **External phase** | | | | | | | | | |
|---|---|---|---|---|---|---|---|---|---|
| Magnesium Stearate | 1,00 | 1,00 | 1,00 | 1,00 | 1,00 | 1,00 | 1,00 | 1,00 | 1,00 |
| Total | 100,00 | 100,00 | 100,00 | 100,00 | 100,00 | 100,00 | 100,00 | 100,00 | 100,00 |

Compositions 1A to 1J were prepared according to the following manufacturing process:
- Mixing of Paliperidone with HPMC K100M geometrically.
- Addition of MCC, Eudragit® RS, SLS and BHT and mixing;
- Kneading with solution of ethanol/water;
- Drying of the granules at 40°C;
- Lubrication of the granules with magnesium stearate;
- Compression to tablets.

The tablet matrix core was prepared with HPMC K100M as hydrophilic/gelling agent and Eudragit® RS as insoluble/hydrophobic polymer.

In order to select the proper ratio of these polymers different amounts were tested having as responses the % drug release at 12h and 24h. The target was to achieve 45-65% drug release at 12h and more than 80% at 24h.

The formulations were tested for their dissolution profiles in USP II apparatus, pH 6.8 + 0.5% tween 80, 150rpm and data of 12h and 24h % drug release were used as responses to determine the proper ratio of hydrophilic and hydrophobic polymer.

According to the obtained results high amount of Eudragit® RS seems to retard the drug release especially when low amount of HPMC is used.

In order to be closer to the target values of drug release after 12h and 24h, the amount of Eudragit® RS had to be limited between 5-10% in combination with amount of HPMC between 20-40%. (Table 2)

**Table 2: Compositions 2A to 2C (not within claimed scope)**

| **Ingredients** | **Trial 2A** | **Trial 2B** | **Trial 2C** |
|---|---|---|---|
| **Internal phase** | % | | |
| Paliperidone | 0,60 | 0,60 | 0,60 |
| Microcrystalline cellulose | 70,35 | 65,35 | 55,35 |
| HPMC K100M | 20,00 | 25,00 | 30,00 |
| Eudragit® RS PO | 5,00 | 5,00 | 8,00 |
| SLS | 3,00 | 3,00 | 3,00 |
| BHT | 0,05 | 0,05 | 0,05 |

| **External phase** | | | |
|---|---|---|---|
| Magnesium Stearate | 1,00 | 1,00 | 1,00 |
| Total | 100,00 | 100,00 | 100,00 |

Compositions 2A to 2C were prepared with the same process as Compositions 1A to 1J.

Compositions 2A-C were tested for their dissolution properties (USP II, pH 6.8 + 0.5% tween 80, 150rpm) and were compared with trial IE which incorporated 10% hydrophobic polymer (Eudragit® RS) in the tablet matrix.

Based on the results, it was decided to maintain the amount of Eudragit® RS to 5% since trials 2A and 2B exhibit the desirable drug release at 12h and 24h whereas amounts higher than 10% suppress the drug release.

The next step of the development was to determine the amount of insoluble coating layer that has to be applied on the tablet matrix core in order to achieve zero order drug release. (Table 3)

Using the core of trial 2A, different amounts of insoluble coating layer were applied (1%-3%) and were tested in dissolution in order to study its effect on drug release.
The insoluble polymer that was used for the coating is Eudragit® RL which is also an ammonio methacrylate copolymer like Eudragit® RS having 10% functional quaternary ammonium groups instead of 5% in Eudragit® RS. These ammonium groups increase the pH-independent permeability of the polymers and thus films prepared with Eudragit® RL are freely permeable to water, whereas films prepared with Eudragit® RS are only slightly permeable to water.

Also a separating layer based on HPMC (Opadry®) was applied between the tablet core and the insoluble coating layer in order to prevent any interaction.

**Table 3: Compositions 3A to 3D (not within claimed scope)**

| | **Trial 3A** | **Trial 3B** | **Trial 3C** | **Trial 3D** |
|---|---|---|---|---|
| **Ingredients** | % | | | |
| Paliperidone | 0,60 | 0,60 | 0,60 | 0,60 |
| Microcrystalline cellulose | 70,35 | 70,35 | 70,35 | 70,35 |
| HPMC K100M | 20,00 | 20,00 | 20,00 | 20,00 |
| Eudragit® RS PO | 5,00 | 5,00 | 5,00 | 5,00 |
| SLS | 3,00 | 3,00 | 3,00 | 3,00 |
| BHT | 0,05 | 0,05 | 0,05 | 0,05 |
| Magnesium Stearate | 1,00 | 1,00 | 1,00 | 1,00 |
| **Total for uncoated tablet** | 100 | | | |

| **1st coating layer** | | | | |
|---|---|---|---|---|
| Opadry® | 3,00 | 3,00 | 3,00 | 3,00 |

| **2nd coating layer** | | | | |
|---|---|---|---|---|
| Eudragit® RL (containing also triethyl citrate) | 1,00 | 1,50 | 2,00 | 3,00 |

Compositions 3A to 3D were prepared with the same process as Compositions 1A to 1J but tablets were further coated with Opadry® (1^{st} coating layer) and Eudragit® RL containing also triethyl citrate (2nd coating layer-insoluble coating layer).

In order to study the effect of different amount of insoluble coating layer, trials 3A-D were tested in pH 6.8, 150rpm without the use of surfactant.

The insoluble coating layer suppressed the dissolution profiles and not proper release was obtained at the target time points as well as at the initial time points.

In order to increase the dissolution profiles, mainly at early time points, it was decided to incorporate in the controlled release layer a small amount of active substance (10% of the labelled amount) so that this amount is dissolved and further absorbed in the small intestine where the permeability of Paliperidone is high. (Table 4)

**Table 4: Compositions 4A to 4D (Not according to the claims)**

| | **Trial 4A** | **Trial 4B** | **Trial 4C** | **Trial 4D** |
|---|---|---|---|---|
| **Ingredients** | % | | | |
| Paliperidone | 0,54 | 0,54 | 0,54 | 0,54 |
| Microcrystalline cellulose | 70,35 | 70,35 | 70,35 | 70,35 |
| HPMC K100M | 20,00 | 20,00 | 20,00 | 20,00 |
| Eudragit® RS PO | 5,00 | 5,00 | 5,00 | 5,00 |
| SLS | 3,00 | 3,00 | 3,00 | 3,00 |
| BHT | 0,05 | 0,05 | 0,05 | 0,05 |
| Magnesium Stearate | 1,00 | 1,00 | 1,00 | 1,00 |
| **Total for uncoated tablet** | 100,00 | 100,00 | 100,00 | 100,00 |
| **1st coating layer** | | | | |
| Opadry® | 3,00 | 3,00 | 3,00 | 3,00 |

| **2nd coating layer** | | | | |
|---|---|---|---|---|
| Eudragit® RL (containing also paliperidone and triethyl citrate) | 1,00 | 1,50 | 2,00 | 3,00 |

Compositions 4A to 4D were prepared with the same process as Compositions 3A to 3D but in insoluble coating layer was further incorporated a small amount of active ingredient.

Dissolution data of trials 4 A-D in pH 6.8, 150rpm showed that when amount of 3% insoluble coating layer was applied the dissolution profile was suppressed resulting in incomplete drug release over 24 hours. Amounts of up to 2% insoluble coating layer resulted in acceptable dissolution profiles since drug release was promoted at early time points.

In order to minimize the drug release in stomach where solubility and permeability of Paliperidone are maximum, an enteric coating layer (using polymer that dissolves in pH>5.5) was introduced over the insoluble coating layer.

Between the two layers a separating layer was also applied so that any interaction is prevented and erosion /dissolution of the insoluble coating layer begins after dissolution of the enteric coating layer.

Formulations based on trial 4B were prepared applying different amount of enteric coating (1-4%) as in Table 5 below.

**Table 5: Compositions 5A to 5D**

| | **Trial 5A** | **Trial 5B** | **Trial 5C** | **Trial 5D** |
|---|---|---|---|---|
| **Ingredients** | % | | | |
| Paliperidone | 0,54 | 0,54 | 0,54 | 0,54 |
| Microcrystalline cellulose | 70,35 | 70,35 | 70,35 | 70,35 |
| HPMC K100M | 20,00 | 20,00 | 20,00 | 20,00 |
| Eudragit® RS PO | 5,00 | 5,00 | 5,00 | 5,00 |
| SLS | 3,00 | 3,00 | 3,00 | 3,00 |
| BHT | 0,05 | 0,05 | 0,05 | 0,05 |
| Magnesium Stearate | 1,00 | 1,00 | 1,00 | 1,00 |
| **Total for uncoated tablet** | 100,00 | 100,00 | 100,00 | 100,00 |

| **1st coating layer** | | | | |
|---|---|---|---|---|
| Opadry® | 3,00 | 3,00 | 3,00 | 3,00 |

| **2nd coating layer** | | | | |
|---|---|---|---|---|
| Eudragit® RL (containing also Paliperidone and triethyl citrate) | 1,50 | 1,50 | 1,50 | 1,50 |

| **3rd coating layer** | | | | |
|---|---|---|---|---|
| Opadry® | 3,00 | 3,00 | 3,00 | 3,00 |

| **4th coating layer** | | | | |
|---|---|---|---|---|
| Kollicoat® MAE 100 (containing also propylene glycol) | 1,00 | 2,00 | 3,00 | 4,00 |

Compositions 5A to 5D were prepared with the same process as Compositions 4A to 4D with the addition of another separating layer -Opadry® between 2^{nd} coating layer (insoluble layer) and 4^{th} coating layer (enteric layer) and an enteric coating layer.

The formulations were tested in dissolution methods in USP II apparatus with 150rpm simulating fasting conditions (pH 1.2 for 2h followed by pH 6.8 for 22h) and fed conditions (pH 4.5 for 4h followed by pH 6.8 for 20h).

Comparing the dissolution profiles it can be concluded that the amount of enteric coating layer has to be up to 3% in order to achieve minimum drug release at acidic environment without decreasing the dissolution rate and extent.

Having concluded to the amount of enteric coating layer and considering the fact that the functional coating layers (enteric and insoluble layer) affect the dissolution profile at the initial time points, trials 6 and 7 were prepared with higher amount of HPMC (30% and 40% respectively) in the tablet core in order to study its effect on drug release after dissolution of the coating layers. (Table 6)

**Table 6: Compositions 6 and 7**

| | **Trial 6** | **Trial 7** |
|---|---|---|
| **Ingredients** | % | |
| Paliperidone | 0,54 | 0,54 |
| Microcrystalline cellulose | 60,35 | 50,35 |
| HPMC K100M | 30,00 | 40,00 |
| Eudragit® RS PO | 5,00 | 5,00 |
| SLS | 3,00 | 3,00 |
| BHT | 0,05 | 0,05 |
| Magnesium Stearate | 1,00 | 1,00 |
| **Total for uncoated tablet** | 100,00 | |

| **1st coating layer** | | |
|---|---|---|
| Opadry® | 3,00 | 3,00 |

| **2nd coating layer** | | |
|---|---|---|
| Eudragit® RL (containing also Paliperidone and triethyl citrate) | 1,50 | 1,50 |

| **3rd coating layer** | | |
|---|---|---|
| Opadry® | 3,00 | 3,00 |

| **4th coating layer** | | |
|---|---|---|
| Kollicoat® MAE 100 (containing also propylene glycol) | 2,00 | 2,00 |

Compositions 6 and 7 were prepared with the same process as Compositions 5A to 5D.

Trials 6 and 7 were tested with USP II apparatus at 150rpm simulating fasting conditions (pH 1.2 for 2h followed by pH 6.8 for 22h) and fed conditions (pH 4.5 for 4h followed by pH 6.8 for 20h) and the dissolution profiles were compared with trial 5B which differs only on the amount of HPMC in the tablet core (20%).

Trials 5B and 7 were also studied in vivo. Based on the in vivo data it was concluded that zero order release mechanism is achieved with both formulations exhibiting Tmax=24h.

Additional trials were performed in which a double amount of API in the insoluble coating layer was introduced in order to increase the dissolution in the early hours (till 4-8h) without affecting the release mechanism in vivo.

Based on tablet core of trial 7 but with 80% API in the core and the rest 20% in the insoluble coating layer, trials 7A-C were prepared applying 1.5%, 2% and 3% of insoluble coating layer respectively. (Table 7)

**Table 7: Compositions 7A to 7C**

| | **Trial 7A** | **Trial 7B** | **Trial 7C** |
|---|---|---|---|
| **Ingredients** | % | | |
| Paliperidone | 0,48 | 0,48 | 0,48 |
| Microcrystalline cellulose | 50,35 | 50,35 | 50,35 |
| HPMC K100M | 40,00 | 40,00 | 40,00 |
| Eudragit® RS PO | 5,00 | 5,00 | 5,00 |
| SLS | 3,00 | 3,00 | 3,00 |
| BHT | 0,05 | 0,05 | 0,05 |
| Magnesium Stearate | 1,00 | 1,00 | 1,00 |
| **Total for uncoated tablet** | **100,00** | | |

| **1st coating layer** | | | |
|---|---|---|---|
| Opadry® | 3,00% | 3,00% | 3,00% |

| **2nd coating layer** | | | |
|---|---|---|---|
| Eudragit® RL (containing also Paliperidone and triethyl citrate) | 1,50% | 2,00% | 3,00% |

| **3rd coating layer** | | | |
|---|---|---|---|
| Opadry® | 3,00% | 3,00% | 3,00% |

| **4th coating layer** | | | |
|---|---|---|---|
| Kollicoat® MAE 100 (containing also propylene glycol) | 2,00% | 2,00% | 2,00% |

Compositions 7A to 7C were prepared with the same process as Compositions 5A to 5D.

Trials 7A to 7C were tested with USP II apparatus at 150rpm simulating fasting conditions (pH 1.2 for 2h followed by pH 6.8 for 22h) and fed conditions (pH 4.5 for 4h followed by pH 6.8 for 20h) and the dissolution profiles were compared with trial 7 which differs only on the amount of active ingredient in the tablet core and the insoluble layer.

**Table 8: Dissolution results of compositions 7, 7A, 7B, 7C under fasting conditions**

| | **2h pH 1.2- pH 6.8, 150rpm % release** | | | |
|---|---|---|---|---|
| **Time (h)** | **Trial 7** | **Trial 7A** | **Trial 7B** | **Trial 7C** |
| 1,0 | 0,45 | 0,47 | 0,70 | 0,63 |
| 2,0 | 0,98 | 1,09 | 0,99 | 0,70 |
| 3,0 | 1,56 | 7,53 | 3,89 | 4,73 |
| 4,0 | 3,57 | 14,14 | 7,07 | 6,71 |
| 6,0 | 6,75 | 21,26 | 15,54 | 9,60 |
| 8,0 | 13,41 | 27,24 | 22,35 | 12,67 |
| 12,0 | 21,24 | 38,71 | 32,15 | 18,74 |
| 14,0 | 28,19 | 44,37 | 38,34 | 24,33 |
| 18,0 | 40,45 | 56,12 | 53,25 | 34,67 |
| 24,0 | 58,11 | 70,25 | 68,51 | 40,25 |

**Table 9: Dissolution results of compositions 7, 7A, 7B, 7C under fed conditions**

| | **4h pH 4.5-pH 6.8, 150rpm % release** | | | |
|---|---|---|---|---|
| **Time (h)** | **Trial 7** | **Trial 7A** | **Trial 7B** | **Trial 7C** |
| 1,0 | 0,50 | 0,29 | 0,36 | 0,26 |
| 2,0 | 0,76 | 0,65 | 1,25 | 0,95 |
| 3,0 | 1,95 | 0,84 | 1,29 | 0,68 |
| 4,0 | 2,71 | 0,96 | 2,35 | 1,25 |
| 6,0 | 7,37 | 11,25 | 12,56 | 6,35 |
| 8,0 | 13,91 | 20,23 | 21,25 | 9,65 |
| 12,0 | 17,93 | 26,54 | 28,65 | 16,54 |
| 14,0 | 26,88 | 36,54 | 35,64 | 19,56 |
| 18,0 | 37,08 | 46,51 | 42,56 | 21,15 |
| 24,0 | 54,67 | 59,64 | 58,65 | 26,56 |

The results of dissolution testing indicate that the presence of higher amount of Paliperidone in the insoluble/controlled release layer enhances the drug release at the early hours and finally the release at 24 hours.

Also, the results confirm that the amount of controlled release layer applied on the tablet core plays a significant role on the drug release.

Trial 7A was also tested in vivo in order to study the effect of the increased amount of API in the controlled release layer. Based on the in vivo data, it is concluded that zero order drug release is achieved.

In order to study the chemical stability of trial 7A, tablets were stored at 25°C/60% RH (normal conditions) and 30°C/65% RH (intermediate conditions) for 6 months and were analyzed in terms of impurities.

**Table 10: Stability data of Composition 7A in normal and intermediate conditions**

| | **STORAGE AT 25°C/60% RH** | | |
|---|---|---|---|
| **SPECIFICATION** | **Zero time** | **3 months** | **6 months** |
| **Total impurities** | 0.50% | 0.49% | 0.56% |
| | | | |

| | **STORAGE AT 30°C/65% RH** | | |
|---|---|---|---|
| **SPECIFICATION** | **Zero time** | **3 months** | **6 months** |
| **Total impurities** | 0.50% | 0.52% | 0.60% |

According to the stability data it can be concluded that the formulation that has been developed is chemically stable after storage in normal and intermediate conditions.

## Claims

1. Paliperidone controlled release tablet in the form of a multi-layered tablet comprising:
a) a matrix core comprising Paliperidone, a hydrophilic swelling polymer and a hydrophobic methacrylic acid copolymer containing low level of quaternary ammonium groups;
b) an insoluble coating layer comprising a methacrylic acid copolymer containing high level of quaternary ammonium groups and Paliperidone;
c) an enteric coating layer comprising an anionic pH dependent polymer.

2. The controlled release tablet of claim 1, wherein the matrix core comprises 20-40% by weight of hydrophilic swelling polymer and 5-10% by weight of hydrophobic methacrylic acid copolymer containing low level of quaternary ammonium groups.

3. The controlled release tablet of claim 2, wherein the hydrophilic swelling polymer is selected from the group consisting of HPMC, HPC, HEC, carrageenan, sodium alginate and xanthan gum.

4. The controlled release tablet of any preceding claim, wherein the hydrophilic swelling polymer is HPMC.

5. The controlled release tablet of claim 1, wherein the insoluble coating layer comprises 10-25% of the labelled amount of Paliperidone.

6. The controlled release tablet of any preceding claim, wherein the insoluble coating layer is comprised in amount 1-2% by weight of the tablet core.

7. The controlled release tablet of claim 1, wherein the anionic pH dependent polymer is a copolymer derived from methacrylic acid/ethyl acrylate.

8. A process for the preparation of an oral controlled release solid dosage form in the form of multi-layered tablet comprising Paliperidone or pharmaceutically acceptable salt thereof as an active ingredient in a matrix core, an insoluble coating layer and an enteric coating layer in order to achieve zero order drug release is provided, which comprises:
- Mixing of Paliperidone with excipients of matrix core comprising a hydrophilic swelling polymer and a hydrophobic methacrylic acid copolymer containing low level of quaternary ammonium groups;
- Kneading with solution of ethanol/water;
- Drying of the granules;
- Lubrication of the granules;
- Compression of granules into tablets;
- Applying a separating coating;
- Applying an insoluble coating layer comprising a methacrylic acid copolymer containing high level of quaternary ammonium groups and 10-25% of the labelled amount of Paliperidone;
- Applying a second separating coating;
- Applying an enteric coating layer comprising an anionic pH dependent polymer.

9. The process according to claim 8, wherein the matrix core comprises 20-40% by weight of hydrophilic polymer and 5-10% by weight of hydrophobic methacrylic acid copolymer containing low level of quaternary ammonium groups.

10. The process according to claim 8, wherein the insoluble coating layer is comprised in amount 1-2% by weight of the tablet core.

## Patentansprüche

1. Paliperidon-Tablette mit kontrollierter Freisetzung in Form einer mehrschichtigen Tablette, umfassend:
a) einen Matrixkern, umfassend Paliperidon, ein hydrophiles Quellpolymer und ein hydrophobes Methacrylsäurecopolymer, das einen geringen Anteil an quaternären Ammoniumgruppen enthält;
b) eine unlösliche Überzugsschicht, umfassend ein Methacrylsäure-Copolymer, das einen hohen Anteil an quaternären Ammoniumgruppen und Paliperidon enthält;
c) eine enterische Beschichtungsschicht, die ein anionisches pH-abhängiges Polymer umfasst.

2. Tablette mit kontrollierter Freisetzung nach Anspruch 1, wobei der Matrixkern 20 bis 40 Gew % hydrophiles Quellpolymer und 5 bis 10 Gew % hydrophobes Methacrylsäurecopolymer umfasst, das einen geringen Anteil an quaternären Ammoniumgruppen enthält.

3. Tablette mit kontrollierter Freisetzung nach Anspruch 2, wobei das hydrophile Quellpolymer ausgewählt ist aus der Gruppe bestehend aus HPMC, HPC, HEC, Carrageenan, Natriumalginat und Xanthangummi.

4. Tablette mit kontrollierter Freisetzung nach einem der vorhergehenden Ansprüche, wobei das hydrophile Quellpolymer HPMC ist.

5. Tablette mit kontrollierter Freisetzung nach Anspruch 1, wobei die unlösliche Überzugsschicht 10 bis 25% der markierten Menge an Paliperidon umfasst.

6. Tablette mit kontrollierter Freisetzung nach einem der vorhergehenden Ansprüche, wobei die unlösliche Überzugsschicht in einer Menge von 1 bis 2 Gew % des Tablettenkerns enthalten ist.

7. Tablette mit kontrollierter Freisetzung nach Anspruch 1, wobei das anionische pHabhängige Polymer ein Copolymer ist, das von Methacrylsäure / Ethylacrylat abgeleitet ist.

8. Verfahren zur Herstellung einer oralen festen Dosierungsform mit kontrollierter Freisetzung in Form einer mehrschichtigen Tablette, umfassend Paliperidon oder ein pharmazeutisch verträgliches Salz davon als Wirkstoff in einem Matrixkern, einer unlöslichen Überzugsschicht und einer enterischen Überzugsschicht um eine Arzneimittelfreisetzung nullter Ordnung zu erreichen, wird bereitgestellt, die umfasst:
- Mischen von Paliperidon mit Hilfsstoffen des Matrixkerns, bestehend aus einem hydrophilen Quellpolymer und einem hydrophoben Methacrylsäure-Copolymer, das einen geringen Anteil an quaternären Ammoniumgruppen enthält;
- Kneten mit Ethanol / Wasser-Lösung;
- Trocknen des Granulats;
- Schmierung des Granulats;
- Komprimierung von Granulat zu Tabletten;
- Aufbringen einer Trennbeschichtung;
- Aufbringen einer unlöslichen Überzugsschicht aus einem Methacrylsäure-Copolymer, das einen hohen Anteil an quaternären Ammoniumgruppen und 10 bis 25% der markierten Menge an Paliperidon enthält;
- Aufbringen einer zweiten Trennbeschichtung;
- Aufbringen einer enterischen Überzugsschicht aus einem anionischen pH-abhängigen Polymer.

9. Verfahren nach Anspruch 8, wobei der Matrixkern 20 bis 40 Gew % hydrophiles Polymer und 5 bis 10 Gew % hydrophobes Methacrylsäure-Copolymer umfasst, das einen geringen Anteil an quaternären Ammoniumgruppen enthält.

10. Verfahren nach Anspruch 8, wobei die unlösliche Überzugsschicht in einer Menge von 1 bis 2 Gew % des Tablettenkerns enthalten ist.

## Revendications

1. Comprimé de Palipéridone à libération contrôlée sous forme d'un comprimé multicouche comprenant :
a) un noyau de matrice comprenant de la Palipéridone, un polymère gonflant hydrophile et un copolymère d'acide méthacrylique hydrophobe contenant un faible niveau de groupements ammonium quaternaire;
b) une couche d'enrobage insoluble comprenant un copolymère d'acide méthacrylique hydrophobe contenant un niveau élevé de groupements ammonium quaternaire et de la Palipéridone;
c) une couche d'enrobage entérique comprenant un polymère anionique dépendant du pH.

2. Le comprimé à libération contrôlée selon la revendication 1, dans lequel le noyau de la matrice comprend de 20 à 40% en poids de polymère gonflant hydrophile et de 5 à 10% en poids de copolymère d'acide méthacrylique hydrophobe contenant un niveau faible de groupements ammonium quaternaire.

3. Le comprimé à libération contrôlée selon la revendication 2, dans lequel le polymère gonflant hydrophile est choisi parmi le groupe constitué de HPMC, HPC, HEC, carraghénane, alginate de sodium et gomme de xanthane.

4. Le comprimé à libération contrôlée selon l'une quelconque des revendications précédentes, dans lequel le polymère gonflant hydrophile est HPMC.

5. Le comprimé à libération contrôlée selon la revendication 1, dans lequel la couche d'enrobage insoluble comprend de 10 à 25% de la quantité indiquée de Palipéridone.

6. Le comprimé à libération contrôlée selon l'une quelconque des revendications précédentes, dans lequel la couche d'enrobage insoluble est présente en quantité de 1 à 2% en poids du noyau du comprimé.

7. Le comprimé à libération contrôlée selon la revendication 1, dans lequel le polymère anionique dépendant du pH est un copolymère dérivé d'acide méthacrylique/acrylate d'éthyle.

8. Un procédé pour la préparation d'une forme posologique orale, solide à libération contrôlée sous forme de comprimé multicouche, comprenant de la Palipéridone ou un sel pharmaceutiquement acceptable de celle-ci comme principe actif dans un noyau de matrice, une couche d'enrobage insoluble et une couche d'enrobage entérique afin d'obtenir une libération d'ordre zéro, est fourni et comprend :
- mélanger la Palipéridone avec les excipients du noyau de la matrice comprenant un polymère gonflant hydrophile et un copolymère d'acide méthacrylique hydrophobe contenant un niveau faible de groupements ammonium quaternaire ;
- pétrir avec une solution ethanol/eau;
- sécher les granules;
- lubrifier les granules;
- compression des granules en comprimés;
- appliquer un revêtement de séparation;
- appliquer une couche d'enrobage insoluble comprenant un copolymère d'acide méthacrylique contenant un niveau élevé de groupements ammonium quaternaire et de 10 à 25% de la quantité indiquée de Palipéridone;
- appliquer un second revêtement de séparation;
- appliquer une couche d'enrobage entérique comprenant un polymère anionique dépendant du pH.

9. Le procédé selon la revendication 8, dans lequel le noyau de matrice comprend de 20 à 40% en poids de polymère hydrophile et de 5 à 10% en poids de copolymère d'acide méthacrylique hydrophobe contenant un niveau faible de groupements ammonium quaternaire.

10. Le procédé selon la revendication 8, dans lequel la couche d'enrobage insoluble est présente en une quantité de 1 à 2% en poids du noyau du comprimé.
